Europäisches Patentamt

European Patent Office

Office européen des brevets

(19)

(11) Publication number: **0 134 622**
**A2**

(12) # EUROPEAN PATENT APPLICATION

(21) Application number: **84303527.0**

(22) Date of filing: **24.05.84**

(51) Int. Cl.⁴: **G 01 N 35/00**
**C 12 Q 1/68**

(30) Priority: **25.05.83 US 497997**

(43) Date of publication of application:
**20.03.85 Bulletin 85/12**

(84) Designated Contracting States:
**AT BE CH DE FR GB IT LI LU NL SE**

(71) Applicant: **LEDLEY, Robert S.**
**1002 Lagrande Road**
**Silver Spring Maryland 20903(US)**

(72) Inventor: **LEDLEY, Robert S.**
**1002 Lagrande Road**
**Silver Spring Maryland 20903(US)**

(74) Representative: **Allam, Peter Clerk et al,**
**LLOYD WISE, TREGEAR & CO. Norman House 105-109**
**Strand**
**London WC2R 0AE(GB)**

(54) **Apparatus and method for separating polynucleotides and detecting specific polynucleotide sequences.**

(57) This invention pertains to a method and apparatus for separating a mixture of polynucleotides and for determining the presence or absence of a specific polynucleotide sequence or sequences in a given mixture of polynucleotides.

EP 0 134 622 A2

Croydon Printing Company Ltd.

1

# APPARATUS AND METHOD FOR SEPARATING POLYNUCLEOTIDES AND DETECTING SPECIFIC POLYNUCLEOTIDE SEQUENCES

## BACKGROUND

### Field of Art

This invention pertains to methods and equipment for the electrophoretic separation of polynucleotide sequences and to methods and equipment for the detection of specific polynucleotide sequences using the technique of DNA-DNA or DNA-RNA hybridization.

### Prior Art

Deoxyribonucleic acid (DNA) and ribonucleic acid (RNA) are key molecules in the hereditary determination of all living organisms. Consequently massive amounts of effort and funding have been and will continue to be expended in the attempt to isolate and identify the exact chemical formulae of various DNAs and RNAs. For background information on the biochemical structure and function of genetic systems in general, see, e.q., Lehninger, Albert L., Biochemistry, Second Edition, Worth Publishers, Inc., New York, N. Y. (1975), particularly chapters 31-35; and

Kornberg, Arthur, <u>DNA Synthesis</u>, W. H. Freeman and Co., San Francisco, CA (1974).

DNA and RNA are comprised of nucleotide chains; a nucleotide chain of any length is referred to as a "polynucleotide". The present invention addresses two specific problems within the broad field of genetics: the separation of a mixture of polynucleotides, and the determination of the presence or absence of a particular polynucleotide sequence or sequences in a given mixture of polynucleotides.

The separation of a mixture of polynucleotides may be accomplished in a number of ways, such as by column chromatography on hydroxylapatite or on an inert matrix (<u>e.g.</u> cellulose) coupled with heparin, acriflavin, oligo-dT, or DNA; by selective gel filtration on carbohydrate gels such as Sephadex$^R$ (Pharmacia Fine Chemicals, Piscataway, N.J.) or acrylamide gels such as BioGel$^R$ (BioRad Labs, Richmond, CA); by density gradient centrifugation in cesium chloride, sulfate, formate, acetate, or trifluoroacetate, preferably in cesium sulfate containing dimethylsulfoxide (DMSO); or by electrophoresis. The preferred technique for the fine separation of multiple polynucleotides is electrophoresis, wherein the mixture of polynucleotides is applied to a porous matrix and separated by size in the presence of an electrical field. The result is a parallel series of bands across the gel matrix at right angles to the direction of current flow, wherein the polynucleotide fragments within each band have identical or very nearly identical molecular weights. <u>See</u>, <u>e.g.</u>, Southern, E., Meth. Enzymol. 68:152-176 (1979). Electrophoretic instruments are commercially available which basically comprise chambers with clamping devices and plugs for attaching the current. The electrophoretic matrix is prepared, loaded with the sample polynucleotide mixture, and clamped in place in the chamber. Electrophoretic buffers must be added manually. Electrophoretic units may be purchased with the power source incorporated in the instrument; otherwise the power source must be purchased separately. Power sources capable of running the system at constant power and high voltage are available but none are temperature feedback regulated. The use of thermostatting platens in electrophoresis is known [see, e.g., Ansorge, W. and L. DeMaeyer, J. Chrom. 202:45-53 (1980); Garoff, H. and W. Ansorge, Anal. Biochem. 115:450-457 (1981); Ansorge, W. and H. Garoff, Electrophoresis '81:635-646 (1981)], and cooling platens using beryllium oxide are known. However, none of the known thermostatting systems are temperature voltage feedback regulated.

The known electrophoretic matrices for the separation of polynucleotides generally comprise polyacrylamide or agarose gels or composites thereof. Polyacrylamide matrices have smaller pores than agarose matrices; therefore when the polynucleotides are relatively large agarose is preferred. Sanger, F. and A. R. Coulson, FEBS Letters 87:107-110 (1978); Ansorge and Garoff, supra; Ansorge and DeMaeyer, supra; and Garoff and Ansorge, supra, all describe the use of very thin (0.01-2 mm) polyacrylamide gels as electrophoretic matrices for polynucleotide separation. However, the use of very thin agarose gels has not been described, presumably because agarose gels less than about 1.5 mm are too fragile to withstand the manual manipulations required in using the prior art electrophoretic equipment.

The second problem addressed by the present invention is that of determining the presence or absence of a particular polynucleotide sequence or sequences in a given mixture of polynucleotides. The generally preferred technique is referred to as hybridization; this technique capitalizes on the natural tendency of polynucleotides to spontaneously pair up with other polynucleotides whose nucleotide sequences are exactly or very nearly exactly complementary. Once the nucleotide sequence of the polynucleotide of interest (the "target") is known, the complementary polynucleotide (the "probe") can be isolated or synthesized. When it is desired to determine whether the target polynucleotide is present in a given mixture of polynucleotides, the probe is added to the mixture. Since the probe is complementary only to the target polynucleotide, only the probe and the target will pair, or hybridize. Detection methods are then used which are specific for the hybridized polynucleotides. The absence of any hybridization indicates that the probe did not "find" any target, and therefore that the target polynucleotide is not present in the mixture. For a general discussion of hybridization theory, see, e.g., Walker, P.M.B., Prog. Nucl. Acid Res. and Mol. Bio. 9: 301-326 (1969); Kornberg, A., DNA Synthesis, W.H. Freeman and Co., San Francisco, CA (1974).

The hybridization technique may be employed in a variety of ways, but one of the most popular is to separate a mixture of polynucleotides by electrophoresis so that the polynucleotides are separated by size in bands within the electrophoretic matrix. Probe is then added to the entire matrix and time is allowed for hybridization (if any) to take place. Unhybridized probe is then washed away and the matrix is examined for the presence of hybridization. Presently known techniques are all performed manually; there is no known apparatus specifically designed for the performance of hybridization techniques.

One standard procedure uses a radioactive probe; hybridization is detected by locating the radioactivity remaining after unhybridized radioactive probe is removed by washing. Alternatively, the polynucleotide mixture may be radiolabelled before electrophoretic separation and non-labelled ("cold") probe added; after washing to remove excess probe an enzyme such as mung bean nuclease or nuclease $S_1$ which preferentially destroys unhybridized polynucleotides is added, leaving only hybridized probe-radioactive target. In either procedure, the presence of radioactively labelled hybridized probe-target polynucleotides must be detected. The traditional methods are procedures known as radioautography and radiofluorography which involve an overlay of photographic or X-ray film which is exposed directly or indirectly by the radioactivity and later developed and analyzed. This procedure is extremely time-consuming, and depending on the nature of the radioactive emitter, the intensity of the radiation, the thickness of the gel, and the sensitivity of the film may require up to several weeks.

The traditional methods of electrophoresis and hybridization are thus time-consuming and labor intensive. The electrophoretic step presently requires preparation and loading of the gel matrix, manual addition of electrolyte, monitoring to regulate temperature and length of the runs, manual washing of the gel in buffers after electrophoresis, and transfer to a drying mode. Presently available hybridization techniques require adding the probe material and soaking or sloshing the gel matrix to insure probe coverage, manual rinsing, and addition of the photographic film. If the gel matrix is relatively thick, the polynucleotides must first be transferred to a thinner matrix, _e.g._ a cellulose nitrate sheet, prior to hybridization. The transfer step is time-consuming and inefficient. _See_, _e.g._, Southern, E., Meth. Enzymol. 68:152-176 (1979). Alternatively, the gel must be dried down to paper-thinness; a method for drying using a blotting procedure is given in Shinnick, T. M., E. Lund, O. Smithies, and F. R. Blattner, Nucl. Acids Res. 2:1911-1929 (1975). This method is also unwieldy and time-consuming (1-1½ hours). The use of ultrathin (0.1-2 mm) agarose gels for electrophoresis and _in situ_ hybridization of polynucleotides has not been described.

It is therefore a purpose of this invention to provide an improved electrophoresis apparatus with provisions for automatic filling and draining of the electrolytic buffer, and a cooling platen and power source with an automatic temperature voltage feedback regulator; along with provisions for an automatic dye front detector and automated washing, acid fixing, and dye bath operations.

It is also a purpose of this invention to provide an improved agarose gel matrix for the electrophoretic separation of polynucleotides and to provide a novel process for the electrophoretic separation and hybridization of polynucleotides using an ultrathin agarose gel matrix.

It is further a purpose of this invention to provide an apparatus suitable for the automated administration of a probe or probes to an electrophoretic matrix or matrices containing separated polynucleotides, with provisions for automated addition and drainage of buffer (i.e., automated wash cycles), automated dye cycle, and automated base cycle for denaturation.

It is yet another object of this invention to provide a novel electronic detection system which is both faster and more sensitive than presently known techniques.

It is further a purpose of this invention to provide an integrated system which combines the novel electrophoretic chamber and the novel hybridization chamber, with provisions for the addition of optional drying, detection, and analysis apparatuses; said novel integrated system to be provided with automated means to move an electrophoretic matrix through all the steps required for polynucleotide separation and/or hybridization and/or detection and analysis.

It is yet another purpose of this invention to provide a process for the electrophoretic separation and/or hybridization of polynucleotides using said novel integrated automated system.

The invention will best be understood by reference to the following specification taken in conjunction with the accompanying drawings in which:

8

FIG. I is a flow diagram summary of the process of the present invention;

FIB. IIA is a plan view of a multiple channel gel handling frame employed in the present invention;

FIG. IIB is a cross-section of the multiple channel gel handling frame taken through A-A';

FIG. III is a side view of a sample slot former;

FIG. IV is an isometric view of a multiple-well sample applicator device for use in conjunction with the multiple channel gel handling frame of FIG. IIA;

FIG. V is a cross-section of the multiple channel gel handling frame containing a gel matrix;

FIG. VI is an isometric view of an apparatus constructed in accordance with the present invention;

FIG. VII is a cross-section of the electrophoresis unit;

FIG. VIII is a cross-section of a suitable microwave/vacuum dryer unit for use in the present invention;

FIG. IX is a cross-section of the hybridization/washing unit;

FIG. X is a block diagram of the detector system.

Referring now to FIG. I, the mixture of polynucleotides 1 to be separated is placed in a suitable matrix 2; the matrix containing the polynucleotide mixture is immersed in a suitable buffer and subjected to an electric field (electrophoresis, 3), resulting in a matrix with the polynucleotides separated in bands by size 4. If the polynucleotides were appropriately labelled prior to electrophoresis, the matrix containing the separated polynucleotides may then be directly detected 7 and analyzed 8. If the polynucleotides contained no label prior to electrophoresis, the matrix containing the separated polynucleotides 4 is subjected to the presence of a suitably selected dye, whereupon the separation pattern may be detected optically and analyzed in order to ascertain the total number and relative positions of the separated polynucleotides. Alternatively, the matrix containing the separated polynucleotides 4 may first be dried and the dried matrix containing the polynucleotides is then dyed, detected, and analyzed, or the dyed matrix may first be subjected to an additional drying step before detection and analysis. Preferably the matrix is dried both before and after dying.

If it is desired that the presence of a particular polynucleotide sequence be detected, the matrix containing the separated polynucleotides 4, with or without having been subjected to dying and/or drying steps as described above, is subjected to the presence of probe 9 and hybridization 5 is allowed to take place. The excess probe 10 is removed by washing with an appropriate buffer 6 and the matrix is examined for the presence or absence of hybridization 7 and the results are analyzed 8. Alternatively, the washed matrix 6 may be dried before detection and analysis. If the polynucleotides have not been stained prior to hybridization 5, they may be dyed after washing 6 by subjecting the matrix to a suitable dye, followed by detection and analysis. Preferably, the dyed matrix is dried before detection and analysis.

Referring now to FIG. II(a) a gel handling frame 100 is provided to support the gel matrix 2 throughout the entire process. Frame 100 is divided into a plurality of parallel channels 102 by a series of parallel ridges 104 running between the channels and along the outermost sides. Channels 102 are aligned in parallel with the eventual electrophoretic current path. The ends of the channels are blocked by removable end dams 106 which are held in place by rubber end guards 108 fastened to the handling frame by screws. FIG. II(b) shows a cross-section of gel handling frame 100 through points A-A', wherein multiple parallel channels 102 are separated by multiple parallel ridges 104. Preferably, the bed of the handling frame is cast in one piece with the ridges 104, of a suitable inert material. Once the gel handling frame 100 has been loaded with the gel matrix 2 (see FIG. V), sample slots 122 may be formed by means of sample slot-forming comb 120.

Referring now to FIG. IV, sample applicator 130 is shown with multiple sample wells 132. In use, sample wells 132 are emplaced over and aligned with channels 102 in gel handling frame 100.

FIG. V shows the loaded gel matrix 2 on optional backing sheet 110 within channels 102 of gel handling frame 100. Once the polynucleotide sample has been applied, the gel matrix surface is optionally covered with protective membrane 114 which remains in place during the electrophoretic process.

Referring now to FIG. VI, an isometric view of the automated instrument capable of separating and detecting polypeptides, lipoproteins and polynucleotides is shown. The instrument includes a plurality of subsystems necessary for performing the aforementioned functions. Electrophoresis unit 200 is emplaced in line with microwave/vacuum dryer 300 which is followed by hybridization/washing unit 400. Each of the aforementioned units is serviced by transport unit 500 which is adapted to carry gel handling frame 100 between each of said subsystems. Gel handling frame 100 rides on two slotted retaining forks 502 mounted on pillow block 504 via rotatable joints 528. Forks 502 engage a pair of small polycarbonate pins at each end of gel frame 100. While not shown, forks 502 are biased downwardly via spring pressure so that they will rotate in a clockwise direction upon retraction of lock arms 522 and 524 by solenoid actuator 520. Block 504 in turn is mounted on rail 506 via a rigid ball-groove shaft (not shown). Lateral movement attains from drive motor 510 slaved to microprocessor 226 (not shown) and is translated to pillow block 504 by chain drive 508. Stepping motor 510 is operable in either forward or reverse directions to enable gel handling frame 100 to be moved back and forth between the various subsystems as required during the process.

Each of units 200, 300, and 400 are mounted on hydraulic drives 202, 302 and 402 respectively. In normal operation, the hydraulic drives for the units not in use are retracted, thereby removing the units from the path of travel of gel handling frame 100. The upper portion 314 of microwave/vacuum dryer 300 is also mounted on an upper hydraulic ram (not shown), which operates in conjunction with lower ram 302.

Referring now to FIG. VI in conjunction with FIG. VII, electrophoresis unit 200 will be described in further detail. Electrophoresis chamber 204 includes body 206 which is in intimate contact with a temperature control platen 208. Connected to temperature control platen 208 are power supply terminals 210 and 212 for the application of DC heating currents thereto. Also contained within body 206 are terminals 214 and 216 which are connected to power supply 218. Terminals 214 and 216 are wire electrodes which are shown schematically in a vertical position in FIG. VII; in actual use terminals 214 and 216 lie horizontally on the bottom of their respective chambers within body 206, perpendicularly to the current flow. Terminals 220 and 222 on power supply 218 are connected to temperature control platen 208 via terminals 210 and 212 respectively. Mounted in the upper surface of body 206 above temperature control platen 208 is temperature sensor 224 which serves as one input to microprocessor 226. Microprocessor 226 is connected to power supply 218 via cable 230 which provides signals to power supply 218 to control the application of power outputs to terminals 210 and 212 and electrodes 214 and 216 respectively.

Also serving as another input for microprocessor 226 is the output of moveably mounted dyefront detector 228. Dyefront detector 228 is a photoelectric reflectance detector to sense the presence thereunder of a dyefront indicator in the gel.

Preferably, within electrophoresis unit 200 there are a plurality of reservoirs follows: Electrolyte buffer reservoir 240, wash buffer reservoir 242, acid reservoir 244, and dye reservoir 246. Each of the aforementioned reservoirs feeds through a respectively associated pump (249, 251, 253, 254) and valving system (241, 243, 245, 247) into inlet pipe 250. Electrophoresis body 206 is provided with drains 254, both of which feed into valve 256 and thence via valves 258, 260, 262 and/or 264 into the respectively associated reservoirs. Each of the aforementioned valves and pumps is connected to and controlled by microprocessor 226. In order not to complicate the diagram, the specific control circuitry is not shown, but will functionally described hereinafter.

The body portion 206 of electrophoresis unit 200 contains an adjustable overflow syphon 211 which maintains a uniform level of electrolyte above gel surface 112.

Referring now to FIG. VI in conjunction with FIG. VIII, microwave/vacuum dryer 300 will be described in further detail. Gel handling frame 100 rests on dryer platform 306 having raised perimeter 310. Upper body 314 comprises can 308 attached to vacuum seal 318 and porous matrix 316 via hose 320. Can 308 fits snugly into groove 312 in base 304 in such a manner that porous matrix 316 rests on or close to gel matrix surface 112. Microwave generator 330 is connected to and controlled by microprocessor 226. The details of microwave/dryer 300 are discussed with more particularity in copending U. S. Patent Application Serial No. 343,256, filed January 27, 1982.

Reference is now made to FIG. VI in conjunction with FIG. IX to provide a description of the hybridization/washing unit 400. Body 408 is constructed much the same as body 204 of the electrophoresis unit. Body 408 supports gel handling frame 100 and preferably provides reservoirs 404 for the various buffers, bases, and acids employed during the hybridization and washing functions. Buffer reservoir 410, base reservoir 412 and dye reservoir 414 are connected via valves 418, 420 and 422 respectively through pump 426 to reservoirs 404. Movably mounted above gel handling frame 100 is a probe dispenser assembly 430. Probe dispenser assembly 430 has a plurality of parallel dispersing heads 432 which are respectively aligned over each of gel channels 102 in gel handling frame 100. Each dispensing head 432 comprises a probe reservoir 444 fitted with exit tube 446 having a one-way valve 442. Reservoir 444 is also fitted with airtight stopper 448 and individual feeder tube 436 which is part of tube assembly 434. Tube assembly 434 is in turn connected to probe pump 440. Probe dispenser assembly 430 is connected by an arm 456 to block 450 which in turn is coupled to belt 452 and stepping motor 454. Microprocessor 226 controls stepping motor 454, valves 418, 420, 422 and 452, probe pump 440, and valves 442.

The preferred detector system will be described with reference to FIG. X (not shown in FIG. VI). A fluorescent screen 606 is placed on top of gel matrix 2. The light that comes from this screen is imaged by means of a large aperture, short focal length quartz lens 602 onto the photocathode of a very high gain microchannel plate 604. The output of microchannel plate 604 is an image on a phosphorescent screen in an integrating secondary electron conduction television camera 608 which is used to view the output of microchannel plate 604. The secondary electron conduction camera 608 has the unique property of being able to integrate an image for a relatively long period (several minutes) with minimum noise introduction. This allows for substantially increased sensitivity in view of the relatively low levels of radioactivity being sensed. The output of camera 608 is an analog signal which is fed via A-to-D converter 610 (8 bit, 256 levels) to frame store 612. The digitized image stored in frame store 612 is a two-dimensional matrix of 512 x 512 picture elements, each element being 8 bits. Once stored in the frame store, the image can be processed, enhanced, and analyzed using well known computational techniques. In order to carry out such subroutines, a digital computer 614 is required. In addition, the terminal associated with digital computer 614 may be used to view images stored in frame store 612 and/or to store them for future analysis.

The preferred process for using the apparatuses of the present invention will now be described in further detail in conjunction with FIGS. I-X.

The mixture of polynucleotides 1 comprises lengths of DNA and/or RNA. Generally, the mixture will be either DNA or RNA and all the DNA or RNA in the mixture will be derived from a single source, i.e. from a single species of microorganism or from a single organism of a higher species. Sources of DNA and RNA and methods of isolation are well known to those skilled in the art. For instance, RNA may be obtained from E. coli, tissue biopsies, blood cells, tumor tissue, RNA viruses, cultured mammalian cells, plant tissues, etc., according to procedures such as those given in, e.g., Gesteland, R.F., and H. Boedtker, J. Mol. Biol. 8:496-507 (1964); Kirby, K.S., Meth. Enzymol. 12(B): 87-99 (1968). DNA may be extracted from cells in any living organism, e.g., blood, amniotic fluid, sperm, biopsy tissue, gut tissue, etc. The DNA extraction process is well-known to those skilled in the art [see, e.g., Marmur, J., Meth. Enzymol. 6:726-738 (1963)].

The purified RNA comprises polynucleotides small enough to be electrophoresed, but the purified DNA comprises very large molecules with complex secondary and tertiary structural characteristics. Therefore the DNA must be reduced to fragments (polynucleotides) small enough to pass through the pores of the matrix. The size range of the fragments is generally approximately 25,000 to 125,000,000 daltons molecular weight (0.1-500 kilobases). In order to reduce the DNA to polynucleotides of the proper size, the DNA is fragmented by either physical or enzymatic cleavage. Physical cleavage ("shearing") is accomplished by hydrodynamic shear or sonication or X-irradiation. See, e.g., Lee, C.S. and C.A. Thomas, Jr., Meth. Enzymol. 29:443-451 (1974); Leadon, S.A. and P.A. Cerutti, Analyt. Biochem. 120:282-288 (1982). Alternatively, the DNA is cleaved by restriction enzymes which act on specific cleavage sites, said cleavage sites being determined by their nucleic acid sequences. Cleavage of a particular DNA sequence by a particular restriction endonuclease always produces the same polynucleotide fragments and therefore is preferable to shearing, which produces random cleavage based on length alone. Examples of restriction endonucleases suitable for use in this invention, are e.g., EcoR I, Hind III, BamH I, etc. Methods of treating DNA with restriction endonucleases to obtain polynucleotides are well known; see, e.g. Maniatis, T., E.F. Fritsch, and J. Sambrook, Molecular Cloning, Cold Spring Harbor Laboratory, pp. 104-106 (1982).

The choice of the appropriate electrophoretic matrix 2 depends on the size of the polynucleotides to be separated. Examples of suitable matrices are gels comprised of from about 0.5 to about 2.0% (w/v) agarose, from about 3.5 to about 20% (w/v) polyacrylamide, combinations of agarose:polyacrylamide (e.g. 0.5% : 2-5% (w/v), and about 1% (w/v) starch; said gels having a thickness of from about 0.1 to about 10 mm. See, e.g., Southern, E., Meth. Enzymol. 68:152-176 (1979). Of these, polyacrylamide and agarose are preferred, and for larger (1 to 100 kilobases) polynucleotides, agarose in the range of from about 0.3 to about 0.6% (w/v) is particularly preferred. Thin gels (0.2-3.0 mm) are preferred for electrophoresis because the electrophoretic process itself generates heat which must be dissipated to prevent the melting of the gel matrix; the thinner the gel the more efficient the heat dissipation. The speed at which the polynucleotides move through the gel matrix is a function in part of the strength of the field; therefore rapid and efficient heat dissipation allows the use of a stronger field (light current) which in turn results in faster separation. The shorter the running time and the faster the separation, the less diffusion occurs within the bands of segregated polynucleotides and the better the resolution of the final product. Therefore thin gels are

0134622

preferred; gels from about 0.2 to about 1.0 mm are particularly preferred.

The gel matrix 2 is prepared and cast on gel handling frame 100 according to procedures well known in the art. See, e.g., Sealey, P.G. and E.M. Southern, in Gel Electrophoresis of Nucleic Acids [D. Rickwood and B.D. Hames, eds.], IRL Press, Oxford, pp. 39-76 (1982). Very thin (0.2 to 3.0 mm) agarose gels are prepared by dissolving 0.5 to 2% (w/v) agarose in a suitable buffer, e.g., 0.04 $\underline{M}$ Tris-acetate, 0.002 $\underline{M}$ EDTA, pH 8.0; 0.08 $\underline{M}$ Tris-phosphate, 0.008 $\underline{M}$ EDTA, pH 8.0; or 0.89 $\underline{M}$ Tris-borate, 0.089 $\underline{M}$ boric acid, 0.002 $\underline{M}$ EDTA, pH 8.0 (the latter two buffers are preferred), and heating at about 95 to about 100°C until the agarose is dissolved. The molten agarose is then poured or rolled on a support which has removable dams set around the edges to prevent the liquid gel from running off the edges. The gel solution is allowed to cool and set, and the end dams are removed. Preferably, the gel matrix is cast on a multiple-channel handling frame 100 such as shown in FIG. IIA. Prior to application of the gel matrix, the ends of channels 102 are blocked by removable end dams 106, which are held in place by metal or plastic guards 108 fastened to the handling frame by suitable fastening means, preferably screws which pass through the guard and into the body of the handling frame. If the gel matrix 2 to be used is less than about 3 mm thick, and if it is desired that the matrix be removed at any time from handling frame 100, a suitable support 110 must be supplied, e.g., a glass or cellulose acetate sheet, or a strong impermeable backing film such as Gel Bond $^{TM}$ (FMC Corp., Marine Colloids Div., Rockland, Maine). The backing material is cut into suitable strips and laid down on the bed of each channel 102. The backing strip is suitably bonded to the handling frame bed (e.g. with water or non-ionic detergent) prior to being overlaid with the gel 2. A precalculated amount of liquid gel matrix is applied to each channel and leveled by tipping or rolling, and the gel is allowed to set and harden.

If sample slots 122 are desired, they may be cut into the hardened gel once it has set. Preferably, sample slots 122 are formed during the setting of the gel by positioning comb 120 across the multiple channels 102 parallel to end dams 106, close to one end of the handling frame 100, and pressing into place. Once the gel matrix 2 has hardened, comb 120 and end dams 106 are are removed. The gel matrix is now cast on the handling frame and ready for use, or the cast gel matrix can be prepared and stored for future use. If the gel matrix is to be stored, it is first overlaid with a protective membrane such as, e.g. cellulose acetate or polycarbonate to prevent drying during storage.

Prior to use, the protective membrane is removed and the sample(s) is (are) loaded on the gel matrix 2. If sample wells 122 have been formed, about 5-20 microliters of sample is pipetted into each well. Larger samples are preferably applied not by means of sample slots but rather by means of a multiple-well sample applicator 130 such as depicted in FIG. IV. Sample applicator 130 is positioned over the gel handling frame 100 in such a way that wells 132 align with channels 102, and the polynucleotide samples to be separated are applied to the wells and allowed to diffuse into the gel. The sample applicator is preferably molded in one piece of a suitable inert material; its construction allows the concentrated application of samples to the gel channels 102 in a consistently repeatable fashion.

The gel matrix is then covered with an electrolyte buffer-wetted membrane 114, preferably of porous cellulose acetate or cellulose nitrate. The entire handling frame, loaded with gel and sample, with an optional backing strip and protective membrane in place (see FIG. V) is then loaded on frame 502 of transport unit 500.

In operation, hydraulic ram 202 is energized and causes electrophoresis unit 200 to rise until gel handling frame 100 is resting on body 206 over temperature control platen 208. Electrolyte buffer is then added to reservoir 206 in an amount sufficient to cover the surface 112 of the gel matrix by about 1-2 mm. The electrolyte buffer may be added by hand; preferably it is added automatically by the following means: When the hydraulic ram reaches the uppermost point of its travel, a signal is sent to microprocessor 226 which in turn opens valve 241 and energizes pump 249 to begin filling electrophoresis body 206 via pipe 250 from electrolyte buffer reservoir 240. At the same time, valve 256 is closed and valve 258 is opened so as to achieve recirculation of the electrolyte between electrophoresis chamber 206 and electrolyte reservoir 240. Valves 260, 262, and 264 are closed. Thus, the level of the electrolyte rises to the top of overflow syphon 211, which overflow then is carried back via pipe 252 and valve 258 into electrolyte reservoir 240. Since the resistance between electrodes 214 and 216 is proportional to the height of the electrolyte in body 206, syphon 211 aids in standardizing the electrophoretic potential from run to run. Overflow syphon 211 also assures that the electrolyte will cover the gel surface by a precise amount, i.e. , 1-2 millimeters.

The electrolyte buffer is selected to provide the proper pH and ionic strength, according to procedures well-known to those skilled in the art; see, e.g., Southern, E., Meth. Enzymol. 68:152-176 (1979). A buffer having the same conductivity as the gel is preferred. An example of a suitable electrolytic buffer is 0.04 M tris acetate, 0.005 M sodium acetate, 0.001 M EDTA, pH 8.0.

While the electrolyte reservoir 206 is filling, power supply 218 is applying potential to contacts 210 and 212 to bring gel matrix 2 up to the desired operating temperature, e.g. about 50-70°C, preferably about 55-60°C. Preferably, electrolyte reservoir 240 is supplied with a heating unit (not shown) to aid in keeping the electrolyte buffer within this temperature range.

When body 206 has been filled to the desired level with electrolyte buffer, microprocessor 226 instructs power supply 218 to energize electrodes 214 and 216 which are lying horizontally at or near the bottom of their respective chambers in body 206, perpendicular to the direction of current flow. During electrophoresis, the buffer is continuously circulated to maintain the proper temperature and pH. In the particularly preferred embodiment, pump 249 is supplied with variable speed settings to allow rapid filling of the chambers within body 206 (e.g. at about 50-100 ml/min) and a slower speed to circulate the electrolyte buffer during electrophoresis (e.g. about 5-10 ml/min).

The temperature of the gel matrix itself is monitored by temperature sensor 224. In FIG. VII temperature sensor 224 is depicted as lying in the upper surface of body 206 over platen 208. Alternatively, temperature sensor 224 may be a movable unit placed directly on the surface 112 of gel 2, preferably in a channel 102 containing the gel without sample and dedicated to this purpose. If circulation of the electrolyte is not adequate to maintain the temperature of the gel (which can rise due to joule heating) within the desired temperature range, the temperature sensor 224 signals microprocessor 226 to reduce the heating current supplied to contacts 210 and 212 to allow platen 208 to cool. Platen 208 is comprised of glass/aluminum or beryllium oxide, preferably beryllium oxide. Temperature sensor 224 is sensitive to changes in temperature of about $\pm$ 1-2°C; in this manner the actual temperature of the gel is maintained within an optimal temperature range which is warm enough to insure that the polynucleotides remain single-stranded, and cool enough to prevent melting of the gel matrix. Furthermore, since the speed of separation and quality of resolution are in part dependent on the amount of voltage applied to the system, control of the joule heating effect in the gel maximizes the allowable voltage while maintaining optimal temperature conditions.

The electrophoresis is allowed to run for a preset period of time. Alternatively, the sample contains a dye indicator such as bromphenol blue or xylene cyanol FF which is specifically chosen for its capacity to traverse the gel at a predictable rate (see, e.g., Maniatis, T., A. Jeffrey, and D.G. Kleid, P.N.A.S. 72:1184 (1955). When the dye reaches a point just beneath dyefront detector 228, the change in color beneath detector 228 is sensed and fed to microprocessor 226 which in turn shuts off power to electrodes 214 and 216. In addition, power to temperature control platen 208 is also terminated, pump 249 is shut off, valve 241 is closed, valve 256 is opened, and the electrolyte buffer is allowed to drain by gravity from electrophoresis body 206 into reservoir 240.

Electrophoresis unit 200 may optionally be fitted with reservoirs containing wash buffer (reservoir 242), dye suitable for dying the sample after electrophoresis (reservoir 246) and/or acid suitable for acid-fixing of the sample after electrophoresis (reservoir 244). The procedure for selecting suitable dyes, acid, and buffers for dying and/or fixing polynucleotide fragments in various gel matrices are well-known to those skilled in the art, see, e.g., Davies, R.W., in Gel Electrophoresis of Nucleic Acids [D. Rickwood and B.D. Hames, eds.], IRL Press, Oxford, pp. 117-172 (1982). Reservoirs 242, 244, and 246 are connected to pumps 251, 253, and 254, fill valves 243, 245, and 247, and drain valves 260, 262, and 264 respectively. These pumps and valves are connected to, and controlled by, microprocessor 226 to allow the automatic filling and draining of electrophoresis body 206 with dye, acid, or wash buffer as desired.

If it is desired to dye the polynucleotide fragments after electrophoresis, microprocessor 226 opens valve 247 and energizes pump 254 to allow the dye to flow into body 206. Valve 256 is closed and valve 264 is opened; drain valves 258, 260, and 262 are closed. When the dye solution has filled the reservoir the excess flows through stand pipe 211 and back into the dye reservoir. Contact of the dye with the polynucleotide fragments in the gel matrix allows the dye molecules to intercolate in the polynucleotide strands and become entrapped. The dye solution is allowed to remain in contact with the polynucleotide strands for about 10 minutes; the microprocessor 226 then shuts valve 247, deactivates pump 254, and opens valve 256, and the dye is allowed to drain by gravity back into dye reservoir 246.

In a like manner, if it is desired to acid-fix the polynucleotide fragments in the gel matrix following electrophoresis, microprocessor 226 opens valve 245, energizes pump 253, opens drain valve 262, and closes drain valves 256, 258, 260, and 264. The gel matrix is saturated with acid for about 10 minutes, or until the polynucleotide fragments are fixed in place within the gel. Microprocessor 226 then closes valve 245, stops pump 253, and opens valve 256, allowing the acid to drain back into reservoir 244 by gravity.

After the gel matrix is exposed to either dye or acid, it is preferably washed with wash buffer supplied in a similar manner via fill valve 243, pump 251, and drain valves 256 and 260. Wash buffer may be circulated through the system or body 206 may be repeatedly filled and drained until residual acid or dye, as the case may be, is removed.

After electrophoresis and the optional subsequent acid fixation and/or dying, the gel matrix is removed from electrophoresis unit 200 and preferably dried. Drying fixes the polynucleotide fragments in the gel matrix to prevent their diffusion and provides a means to rapidly equilibrate the gel matrix upon treatment with various solutions. The dry gel acts like a blotter, rapidly absorbing any applied solution and partially reinflating the matrix.

If supportive backing 110 was used, the drying may be done with the gel attached to the backing but removed from the handling frame 100. If no backing was used, the gel matrix is preferably dried while still in the frame. In either case it is preferable to have a protective membrane 114 in place during drying to prevent lateral shrinkage and cracking. Any suitable drying means may be used, e.g. blotting of the gel surface followed by evaporation at room temperature, dry heat, or vacuum. Preferably, the drying means is microwave-vacuum dryer unit 300: at termination of the operation of electrophoresis unit 200, hydraulic ram 202 is lowered so that the electrophoresis unit no longer interferes with the travel of block 504 and arms 502 which support gel handling frame 100. Microprocessor 226 then provides an input to stepping motor 510 which causes block 504 to be incremented into position over microwave/vacuum dryer 300. Upon block 504 reaching a position juxtaposed to microwave vacuum dryer 300, solenoid actuator 520 is energized thereby causing arms 522 and 524 to move outwardly about hinge 526 so as to cause arms 502 to disengage from the pins which extend outwardly from gel handling frame 100. Subsequently, ram 302 is energized causing base member 304 of the microwave/vacuum dryer to elevate. As tray portion 306 of base 304 engages the bottom of gel handling frame 100, tray 306 begins to move upwardly with base 304. At such time arms 502 are engaged by raised portion 310 and are caused to pivot upwardly about axes 528 and out of engagement with gel handling frame 100. Base portion 304 continues upwardly until slot 312 engages to the bottom edge of can 308. At such time, the upper surface of gel handling frame 100 comes into contact with a porous matrix 316 which adheres to the bottom of a rubber vacuum seal 318. Hose 320 leads to a source of vacuum which is energized on operation of the microwave/vacuum dryer. The porous matrix is fritted glass or a polyethylene sheet (preferred). Preferably the entire drying chamber is completely metal shielded to prevent microwave leakage.

Subsequently, a mild vacuum is applied via vacuum hose 320 to the gel and the microwave heater unit 330 is energized so as to cause a vacuum dessication and drying of electrophoresis gel 2. Microprocessor 226 controls the operation of this system, preferably causing intermittent operation of the microwave generator in response to the operation of microswitches which are engaged when the vacuum dryer is in its fully assembled state (not shown).

The drying of the gel is preferably performed by vacuum dessication at moderate heat. Thin gel can be fully dried within about 10 minutes. The gel frame 100 when in final position is essentially sealed under a fixed manifold comprising the close-fitting array of porous matrix 316 covered by vacuum sealing damn 318 which is in turn connected to a vacuum system via vacuum hose 320. These materials are inert to microwaves and the aqueous gel can be heated by the microwave generator.

At termination of operation of microwave/vacuum
dryer 300, ram 302 retracts, lowering base unit 304.
Gel handling frame 100 may then be removed manually.
Preferably tray 100 is removed automatically by
microprocessor 226 causing arms 502 to engage frame
100 by the retraction of arms 522 and 524 as a result
of deactuation of relay 520. After gel handling frame
100 is engaged with carrier 504, motor 510 is energized to cause the entire assembly to move away from
base unit 304.

If the polynucleotide fragments have been dyed,
their separation may be analyzed by direct visible
inspection. Alternatively, if the polynucleotide
fragments have been radiolabelled, they may be analyzed as described hereinafter. Electrophoresis
allows determination of polynucleotide fragments
present in the sample, separated in bands by molecular
weight.

If it is desired to determine whether or not a
specific polynucleotide sequence is present, the
additional step of hybridization is undertaken. A
probe polynucleotide sequence is prepared which is
complimentary to the target polynucleotide sequence of
interest. Where the target polynucleotide is an RNA
fragment, the complimentary probe sequence may be
prepared by direct synthesis according to methods
known to those skilled in the art, see, e.g. Edge,
M.D., et al., Nature 272:756 (1981), or, alternative-
ly, by reverse transcription. Isolated target RNA is

used as a template in the presence of deoxyribonucleoside-5'-triphosphates, a primer with a free 3'-hydroxy terminus, and RNA-directed DNA polymerase (reverse transcriptase) to yield a DNA sequence which is complementary to, and hybridized with, the target RNA sequence. Denaturation separates the DNA from the RNA, followed by treatment with RNAse which destroys the RNA and leaves the DNA sequence to be used as a probe. See, e.g., Harrison, T.R., G.D. Birnie, A. Hell, S. Humphries, B.D. Young, and J. Paul, J. Mol. Biol. 84:539 (1974). Where the target polynucleotide sequence is a DNA fragment, the complimentary probe sequence may be prepared by direct synthesis according to methods known to those skilled in the art, see, e.g., Edge, M.D., et al., Nature 272:756 (1981), or, alternatively, by a process known as nick translation. Target duplex DNA is nicked with DNAse and subjected to the presence of appropriately labelled deoxyribonucleoside-5'-triphosphates, DNA polymerase, and DNA ligase. The nicks are filled in with the labelled deoxyribonucleoside-5'-triphosphates, yielding intact labelled duplex DNA. Denaturation at about 100°C for about 10 minutes yields a solution of single-stranded labelled DNA suitable for use as probe. See, e.g. Maniatis, T., E.F. Fritsh, J. Sambrook, P.N.A.S. 72:1184. Of course, if unlabelled probe is desired, isolated target DNA is simply denatured to yield a suitable probe solution. See discussion below on labelling of probe.

It is well understood by those skilled in the art that in order for hybridization to take place, the target and the probe must be single-stranded. Isolated RNA is usually single-stranded, but isolated DNA in its natural form is usually double-stranded.

Therefore at some point prior to hybridization, the target double-stranded DNA must be unwound into single-stranded pieces by a process called "denaturation" which is conceptually the reverse of hybridization. This may be accomplished in one of three ways. In the first method the double-stranded DNA is denatured, followed by fragmentation of the single strands, which single-stranded fragments (polynucleotides) are electrophoresed, then hybridized. In the second method the DNA is first fragmented and the fragments are denatured, resulting in single-stranded fragments (polynucleotides) which are then electrophoresed and hybridized. In either of these methods denaturation is generally accomplished either by treatment with alkali, e.g. 1 M sodium or potassium hydroxide at a pH of about 13 to 14, or by exposure to heat at about 60 to about 70°C, or both. See, e.g., Shinnick, T.M., E. Lund, O. Smithies, and F.R. Blattner, Nucl. Acids Res. 2:1911-1929 (1975).

In the third method the DNA is fragmented and the double-stranded fragments are electrophoresed, followed by denaturation by immersion of the entire matrix containing the separated DNA fragments in alkali such as 1 M sodium or potassium hydroxide at a pH of about 13 to 14 for 15-60 minutes at room temperature; or by treatment with heat at about 60-70°C; or both. The first two methods of denaturation are preferred; fragmentation followed by denaturation prior to electrophoresis is particularly preferred.

If single-stranded DNA is left in contact with its natural complementary strands, it will tend to spontaneously reanneal (self-hybridize) back to double-stranded form. Therefore once the DNA has been denatured, care must be taken to prevent spontaneous reannealing. Reannealing is favored by moisture, high ionic strength, and moderately elevated temperatures (see discussion below.) Therefore, denatured DNA is generally best preserved by storage at temperatures of about 4°C to about 40°C in buffers of low ionic strength, e.g. 1 X SSC (0.15 $\underline{M}$ NaCl, 0.15 $\underline{M}$ sodium citrate, 0.002 $\underline{M}$ EDTA, pH 7.0). Denatured DNA in the matrix may be prevented from reannealing by drying, as polynucleotides cannot move freely through the matrix in the absence of a liquid phase.

Effective hybridization requires not only that the target polynucleotide sequence be single-stranded and that the probe sequence be single-stranded, but that the target and probe be of a sufficient length to allow effective interchain bonding. Generally, the target and probe must each be at least about 10 bases in length; preferably the target is from about 0.1 to about 1 kilobase long, and the probe is about 0.1 to about 20 kilobases in length. These relatively large polynucleotide sequences are generally obtained by restriction digest with enzymes such as those described above under sample preparation; because of their size these fragments do not travel well by diffusion in relatively small-pored matrices such as polyacrylamide. Therefore, agarose gels are preferred for hybridization; particularly preferred is a matrix of about 5.0 to about 2.0% (w/v) agarose.

If the agarose gel matrix is relatively thick, i.e. more than about 5.0 mm thick, the polynucleotide fragments are embedded deeply in the matrix after electrophoresis and the efficiency with which the probe binds to the target is severely reduced. To insure effective hybridization, the target polynucleotides must first be lifted from the gel matrix and transferred to a suitable non-interfering matrix without disturbing their relative positioning. E. Southern has devised a technique ("Southern blot technique") wherein the gel matrix containing the separated polynucleotide fragments is sandwiched between a bottom layer of absorbent toweling containing buffer and an upper layer of cellulose nitrate sheeting, on top of which is placed more absorbent dry toweling. As the top dry toweling absorbs moisture, buffer is drawn from the bottom wet toweling, up through the gel matrix and into the cellulose nitrate sheeting by wick action, carrying the individual bands of separated polynucleotide fragments with it. The polynucleotide fragments are thus each transposed vertically onto the cellulose sheeting, where they become entrapped. The toweling is then removed, the polynucleotides are fixed by vacuum drying, and the sheeting containing the fragments is used as a hybridization matrix. See Southern, E., Meth. Enzymol. 68:152-176 (1979). The disadvantages of this process are that the transfer of the polynucleotides to the cellulose nitrate matrix is 1) inefficient, as many fragment bands are incompletely transferred and much sample is lost, and 2) very time-consuming, requiring from about 3 to about 24 hours.

It is therefore preferred that the agarose gels be thin enough so as not to interfere with hybridization, thus allowing for hybridization to take place within the electrophoresis gel itself and avoiding the necessity for a transfer for a separate hybridization matrix. See, e.g., Shinnick, T.M., E. Lund, O. Smithies, and F.R. Blattner, Nucl. Acids Res. 2:1911-1929 (1975). Preferably, the agarose gel comprises from about 0.1 to about 1% (w/v) agarose and is from about 0.2 to about 1.5 mm thick. Agarose gels this thin are very fragile and require support if they are to be moved or handled. This support is provided by, e.g. handling frame 100 and/or by support backing sheet 110.

Hybridization is performed by exposing the thin agarose gel matrix to the probe material. This may be done manually, but preferably is performed by hybridization/washing unit 400, as follows:

Microprocessor 226 directs gel handling frame 100 to be carried by arms 502 and block 504 to a position directly above body 403. Hydraulic ram 402 is actuated causing body 403 to elevate and to engage gel handling frame 100 containing the electrophoresed polynucleotides in gel matrix 2. Preferably, gel matrix 2 is covered with protective membrane 114 throughout the hybridization process. Simultaneously, valves 442 are opened and pump 440 is actuated to pump air into sealed reservoirs 444 via tube assembly 434 and individual feeder tubes 436. Shortly thereafter (to enable the probes to reach the ends of exit tubes 446), stepping motor 454 is energized causing arm 456 to begin traversing the length of gel handling frame 100. The probe material is thus pumped and distributed along the center of the gel channels 102 via movement of arm 456. The same probe may be applied to all channels, or alternatively a different probe may be applied to each channel. Once probe dispenser 430 has reached the limit of its travel, microprocessor 226 shuts off probe pump 440 and closes valves 442, and probe dispenser 430 is incremented back to its home position. If the gel matrix was previously dried, contact with the probe solution causes the gel to reinflate. Methods for the selection of suitable buffers, temperatures, and concentrations are well-known to those skilled in the art; see, e.g. Maniatis, T., E.F. Fritsch, J. Sambrook, Molecular Cloning, Cold Spring Harbor Laboratory, p. 156 (1982); Shinnick, T.M., E. Lund, O. Smithies, and F.R. Blattner, Nucl. Acids Res. 2:1911-1929 (1975); Southern E., Meth. Enzymol. 68:152-176 (1979).

The probe material is allowed to rest in contact with the single-stranded polynucleotides in the gel matrix until hybridization is complete. The hybridization rate is a function of temperature, pH, ionic strength of the probe solution, and the specific nucleotide composition of the target and probe polynucleotides, and can be calculated according to methods well-known to those skilled in the art; see, e.g. Britten, R.J. and D.E. Kohne, Science 161:529-540 (1968); Marmur, J. and P. Doty, J. Mol. Biol. 5:109 (1962). With ultrathin gels, hybridization is allowed to take place for about 1 to about 4 hours, preferably for about 1 to about 2 hours. The use of ultrathin gels minimizes the required diffusion time to one half or less than that required for thicker gels.

After a suitable interval, valve 418 is opened, pump 426 energized, and wash buffer from reservoir 410 is pumped into reservoir 404. Valve 452 is closed and reservoir 404 is allowed to fill until the buffer covers the surface of gel matrix 2 to a depth of about 1-2 mm. Excess buffer flows out through standpipe 406 and into waste reservoir 450. Buffer is circulated in this matter for a suitable interval to allow all excess probe to be removed. Thereafter microprocessor 226 closes valve 418, stops pump 426, and opens drain valve 452, allowing the remaining buffer to drain by gravity from reservoirs 404 via drains 460 to waste reservoir 450.

Preferably, the gel matrix is then dried. Ram 402 is lowered and gel handling frame 100 is transported via transport unit 500 back to microwave/vacuum dryer unit 300, and the gel is dried as before. Preferably the wash/dry cycle is repeated 3-4 times. Without intermittent drying, this cycle would take about 2 hours, but by drying and reinflating the gels, the washing can be completed in less than one hour. It is particularly preferred that the gel is washed and dried several times in succession, ending with a dried gel which is ready for detection and analysis.

Hybridization/washing unit 400 is also preferably equipped with optional reservoirs 412 and 414 containing base suitable for denaturation and dye for staining the polynucleotide fragments, respectively. As described above, it is imperative that the target polynucleotide be in single-stranded form (i.e., denatured) prior to hybridization. If the target polynucleotides have been electrophoresced in double stranded form, they must first be denatured prior to hybridization; if the target polynucleotides have been electrophoresced in single-stranded form, it may be desired to insure complete denaturation after electrophoresis by repeating the denaturation process. The separated polynucleotide fragments may be denatured (or redenatured) in situ within gel matrix 2 in the hybridization/washing unit 400 prior to hypbridization. In a manner similar to that described above for the wash buffer, base is pumped from reservoir 412 through valve 420 by pump 426 and allowed to fill reservoirs 404. Suitable bases for denaturation are known to those skilled in the art, e.g. warm (40°C) 1 M KOH. The polynucleotides are allowed to sit in contact with the circulating base until all the polynucleotide fragments are completely denatured, generally for about 3 to about 10 minutes. The remaining base is then drained into waste reservoir 450 and the gel is washed and dried as described above.

After hybridization, the polynucleotide fragments may be dyed with a suitable dye, e.g. acridine orange or ethidium bromide. Preferably, hybridization/washing unit 400 is equipped with dye reservoir 414 and valve 422 to allow the polynucleotide fragments to be bathed in dye in a process analogous to those described above for application of wash buffer and base. After such a dye step the gel is preferably washed and dried as before.

The gel matrix containing the electrophoresed and hybridized polynucleotide fragments is now ready for detection and analysis. The polynucleotide fragments in the matrix are invisible to the naked eye; the addition of an intercolating dye as discussed above renders the polynucleotides, hybridized and non-hybridized, visible under ultraviolet illumination. In order to detect which (if any) polynucleotides have undergone hybridization, a detectable label is provided to differentiate the hybridized polynucleotides from the non-hybridized polynucleotides present.

As stated above, hybridization involves the binding of a single-stranded target polynucleotide fragment with its complementary probe polynucleotide sequence. The target polynucleotide may be reduced to a single-stranded state (denatured) before or after electrophoresis but denaturation must take place before hybridization with a probe polynucleotide sequence can begin. Either the target or the probe must be labelled prior to hybridization with a radiative energy-emitting marker selected for its ability to be detected in low amounts, e.g. radioactivity or fluorescence, according to procedures well-known to those skilled in the art. Preferably, the single-stranded non-labelled target polynucleotide is hybridized with a labelled probe; after rinsing away excess probe only the band or bands which have undergone hybridization will retain the label. Alternatively, the entire polynucleotide sample containing the target fragments is labelled prior to electrophoresis and is then hybridized with a non-labelled probe polynucleotide sequence. Non-hybridized single-strand sample polynucleotide fragments (which are also labelled) are then degraded by treatment with nucleases which are selective for single-stranded polynucleotides (e.g. mung bean nuclease or nuclease $S_1$) and washed away, leaving only the hybridized, double-stranded fragments in the matrix. There are then detected by virtue of the label present in the target strand. For a summary of labelling techniques and processes, see, e.g. Davies, R.W., in Gel Electrophoresis of Nucleic Acids [D. Rickwood and B.D. Hames, eds.], IRL Press, Oxford, pp. 117-172 (1982).

Radioactive label may then be detected by a process known as radioautography or radiofluorography, wherein a layer of photographic or X-ray film is laid on the gel matrix and the film is left in place to be exposed by the radioactivity emanating from the site(s) of the label. The exposure time required is dependent in part on the concentration and specific activity of the label, the thickness of the gel, and the sensitivity of the film, and generally takes from about 4 hours to about 3 weeks. See, e.g. Bonner, W.M. and R.A. Laskey, Eur. J. Biochem. 46:83-88 (1974); Randerath, K., Anal. Biochem. 34:188-205 (1970).

Therefore it is preferred that the automated detection means provided for as described in Figure X be employed. After hybridization, washing, and drying are complete, microprocessor 226 activates transport unit 500 to engage gel handling frame 100 via arm 502; ram 302 is lowered and can 314 is raised until gel handling frame 100 is clear of microwave/vacuum dryer unit 300, and frame 100 is transported to detector system 600. Dried gel matrix 2 containing the labelled hybridized polynucleotide fragments is disposed parallel to and in contact with fluorescent screen 606. An example of a suitable fluorescent screen is DuPont Chronex Lightning Plus$^{TM}$, a calcium tungstate screen which peaks at approximately 400nm. The loci of radioactivity, present as discrete bands within ultrathin dried gel matrix 2, are detected by phosphorescence lumination. The radioactive emissions from the gel interact with the phosphor on screen 606 to produce photons. The photons are focused by large aperture, short focal length quartz lens 602 (e.g. Canon, Inc., Tokyo, Japan) onto microchannel plate 604, which amplifies the signal. Suitable microchannel plates are, e.g. VARO Model 6300-1 (Varo,

Inc., Garland, TX) or CEMA 3810 (Galileo Electro-Optics Corp., Sturbridge, Mass.). The output of microchannel plate 604 is recorded by integrating secondary electron conduction television camera 608 (e.g. Westinghouse Type #WX625 with a secondary electron conduction tube Type #WX30893, Westinghouse Corp., Elmira, N.Y.). The output of camera 608 is sent to A-to-D converter 610 (e.g. TDC 1007J, TRW Corp., Redondo Beach, CA) and the image is stored in digital frame store 612 (e.g. 274 D, Colorado Video, Boulder, Colo.). The stored image can then be processed by the digital computer 614. The result is a two-dimensional digital image of the pattern of radioactivity in the hybridized bands.

A fluorescent label is detected in a similar manner, except that the energy emissions are already in the form of photons and the use of phosphor screen 606 is eliminated. If the fluorescent label has a high enough specific activity, it may be possible to directly visualize the fluorescence without the use of microchannel image intensifier 604. Methods of labelling polynucleotides with fluorescent label are well-known to those skilled in the art. Preferably, the polynucleotides are labelled with a fluorescent marker such as, e.g., Enzo Bio-Probe$^{TM}$ (Enzo Biochem, Inc., New York, NY) in a manner analagous to that of Hutchinson, N.J., P.R. Langer-Safer, D.C. Ward, B.A. Hamkalo, J. Cell Bio. 95:609-618 (1982).

The detector system is sensitive to individual events and the use of ultrathin gels minimizes absorption of signal by the gel itself. The result is sharper resolution than that achieved by the prior art. Further, the sensitivity of the detector system allows the use of exceptionally low specific activity in the label relative to that required by the prior art techniques.

The presence or absence of the target polynucleotide in a particular mixture of polynucleotide fragments is confirmed by the presence or absence of hybridization with the selected probe, as determined by the detection techniques discussed above. Preferably, a standard sample known to contain standard target polynucleotide sequence(s) is run concurrently as a control. Where further information is desired, such as the concentration and/or specific activity of the hybridized polynucleotides, or the molecular weight of electrophoresed polynucleotides, or the distance of electrophoretic migration relative to the starting point or relative to a given control, the use of a computer imaging and analysis system such as that shown in U. S. Patent No. 4,229,797 (e.g. Digital Equipment Corp., Maynard, Mass.) is preferred. The use of a multiple-channel gel handling frame reduces distortion and shrinkage of the ultrathin gel matrix and allows accurate computer analysis based on information provided by the above standard, as opposed to the use of the more traditional large single-unit gel matrix (containing multiple parallel series of separated polynucleotide bands) which increases the risk of local band boundary distortion and requires trained human evaluation and analysis.

It is to be understood that the apparatus of the present invention, or subsystems thereof, may be used for the separation of biopolymers other than polynucleotides. As used hereinabove and below, the term "biopolymers" means polynucleotides, polypeptides, and lipoproteins. In particular, persons skilled in the art know how to adapt gel matrix 2 and the electrophoretic buffers, wash buffers, acids, and dyes described above in conjunction with electrophoresis unit 200 to allow said electrophoresis unit to be used for the electrophoretic separation of polypeptides and lipoproteins.

A further understanding of the invention can be had from the following representative examples. Unless otherwise specified, all operations were performed at ambient temperature (20-25°C) and one atmosphere pressure.

## Example 1

### Preparation of DNA for Electrophoresis

DNA Standard:

A sample of duplex DNA containing 8 fragments (0.13-23.5 kilobases) of lambda-DNA generated by treatment with Hind III restriction enzyme was obtained from Bethesda Research Labs Inc. (Gaithersburg, MD). An aliquot containing 12 µg (19 µl) was mixed with 31 µl of electrophoresis buffer (0.08 M Tris-phosphate, 0.008 M EDTA, pH 8.0) and heated to 65°C for 10 minutes to denature the DNA. The sample was then cooled in ice and mixed with 10 microliters of solution containing 2 g sucrose and 12.5 mg bromphenol blue (dyefront marker) in 5 ml total volume.

Each 10 µl sample contained 2 µg DNA Hind III fragments.

DNA Sample:

Samples of DNA to be electrophoresced are isolated according to the method of Marmur, J., Meth. Enzymol 6:726-738 (1963) and treated with restriction endonucleases as described in Maniatis, T., E.F. Fritsch, and J. Sambrook, Molecular Cloning, Cold Spring Harbor Laboratory, pp. 104-106 (1982).

The resulting duplex polynucleotides are then treated as above to provide samples suitable for electrophoresis.

## Example 2
### Preparation of RNA For Electrophoresis

RNA is isolated according to the procedure described in Gesteland, R.F. and H. Boedtker, J. Mol. Biol. 8:496-507 (1964) or Kirby, K.S., Meth. Enzymol. 12(B):87-99 (1968). The purified RNA is depurinated in 0.2 mM HCl, washed, and denatured in 1M NaOH or KOH. After neutralization, the sample is suspended in electrophoretic buffer containing sodium dodecyl sulfate, formaldehyde, methyl mercury hydroxide, and glyoxal as denaturing agents and electrophoresis is run under denaturing conditions. For RNA electrophoresis the agarose gel solution is also made up in the electrophoretic buffer containing the denaturing agents.

## Example 3
### Preparation of Agarose

150 mg agarose (Sea Kem HGT, FMC Corp., Marine Colloids Div., Rockland Maine) was added to 25 ml electrolyte buffer (0.08 $\underline{M}$ Tris-phosphate, 0.008 $\underline{M}$ EDTA, pH 8.0) and heated to boiling for 5 min. to dissolve the agarose. The clear solution was cooled to 65°C and the volume was readjusted to 25 ml by adding distilled water. This procedure yielded a 0.6% (w/v) solution of melted agarose.

43

## Example 4
### Backing Materials

One gram of agarose [Sea Kem HGT, FMC Corp., Marine Colloids Div., Rockland, Maine] was made up to 100 mls. in water and dissolved at 95°C. A layer of agarose approximately 2-5 mm thick was poured on 3x5 cm squares of various support materials and allowed to set. The solidified gel was then tested for bonding to the support material by horizontal swishing in water for approximately one-half hour and by vertical immersion in a beaker of water with gentle swirling. The results are presented in Table I:

TABLE I

ADHESION OF THIN AGAROSE GELS

TO VARIOUS SUPPORT MATERIALS

| Support Material | Adherence | |
|---|---|---|
| | Horizontal | Vertical |
| Cellulose acetate[1] | Excellent | Excellent |
| Gel Bond TM [2] | " | " |
| Gel Fix TM [3] | " | " |
| Polycarbonate [4] | Fair | Fair |
| Polyvinylchloride [4] | Poor | Poor |
| Polystyrene [4] | Excellent | Excellent |
| Glass plate (untreated) | Poor | Poor |

[1] Helena Labs, Beaumont, TX
[2] FMC Corp., Marine Colloids Div., Rockland, Maine
[3] Serva Fine Chemicals, Garden City Park, N. Y.
[4] Read Plastics Corp., Rockville, Md.

## Example 5
### Drying

A. The support materials of Example 4 (without agarose) were heated in a microwave/vacuum dryer for 3 minutes at cook mode. No charring or evidence of brittleness or decomposition was noted.

B. A 1% (w/v) agarose gel prepared as described in Example 4 was cast 3 mm deep on a 7x10 cm piece of Gel Bond$^{TM}$ and allowed to solidify. The cast agarose gel and support were dried in a microwave gel drier (Sears Roebuck Model 564) for about 10 minutes in the defrost mode, with the on/off cycling of the line current at 4.5 secs. on, 9 secs. off (total time: 30 minutes). The film dried clearly, without tearing or distortion, and adhered well.

The dried film was then reinflated by overlaying about 70 mls of water. The gel reinflated almost instantly but did not attain its original volume.

C. One per cent (w/v) agarose in water was cast on three 7x10 cm Gel Bond sheets to depths of 0.75, 1.5, and 3.0 mm respectively and allowed to set. The cast gels were then dried on their backings in a microwave dryer under vacuum in cook mode for 9 minutes, with the on/off cycling of the line current at 4.5 secs. on, 9 secs. off (total time: 27 minutes).

The thickest gel (3 mm) dried in 9 minutes. The Gel Bond sheets showed no signs of cracking, yellowing or deterioration.

## Example 6
## Preparation of Agarose Matrix
## In Gel Handling Frame

End dams 106 are affixed to frame 100 with guard 108 to form a series of shallow water-tight troughs. The assembled unit is placed on a level surface maintained at 65°C. Melted agarose (Example 3) is added using a warmed pipet. Each channel 102 requires 1.5 ml melted gel solution per mm thickness of the desired gel matrix. In casting gels greater than 1 mm thick, the melted gel will flow freely. In casting gels less than 1 mm thick, the melted agarose must be spread in a thin film over the surface to overcome its beading tendency. Comb 120 is inserted to form sample wells and the level surface is cooled, causing the agarose to gel.

Comb 120 and guards 108 and dams 106 are then removed. The entire surface of the gel is covered with a porous membrane 114 prewetted with electrolyte buffer (0.08 $\underline{M}$ Tris-phosphate, 0.008 $\underline{M}$ EDTA, pH 8.0) and gently patted to provide intimate contact with all surfaces.

Slits are cut into membrane 114 over the sample wells with a scalpel fitted with a #11 blade. The DNA samples (Example 1) are added to the sample slots using a micropipet.

## Example 7
## Electrophoresis

A. The gel frame containing DNA samples (Example 6) is placed in body 206 of the electrophoresis unit 200. The electrolyte chambers (250 ml capacity) in body 206 fill with electrolyte buffer (0.08 $\underline{M}$ Tris-

phosphate, 0.008 $\underline{M}$ EDTA, pH 8.0) from reservoir 240. Stand pipe 211 is set to maintain a 0.2 cm layer of buffer over the gel surface. Valves 241 and 258 open, valve 256 closes, and pump 249 delivers 5 ml buffer per minute to produce a gentle flow of electrolyte over the surface.

Electrophoretic separation is accomplished by establishing a potential difference of 1000V (0.1 Amp) across electrodes 214 and 216. The temperature is maintained at 65°C via temperature control platen 208 in conjunction with the use of prewarmed electrolyte buffer from reservoir 240 and the use of thermosensor 224 (which triggers microprocessor 226 to regulate the power from supply 218 to terminals 214 and 216 to control Joule heating).

When electrophoresis has proceeded for 2 hours, the dye indicator in the sample will have moved approximately 7 cm from the origin. This is detected optically by sensor 228 and relayed to microprocessor 226 which terminates electrophoresis by shutting off power supply 218 and pump 249. Valve 241 is closed and valve 256 is opened to allow the electrolyte to drain back to reservoir 240.

B. Valves 258 and 256 then close, valve 247 opens, and pump 254 delivers a solution of ethidium bromide (500 mg/l) (Sigma Chemical Co., St. Louis, MO) to the unit. After 10 minutes, pump 254 shuts off, valve 247 closes, valves 264 and 256 open, and the chambers are drained.

The gel containing the electrophoresed DNA is dried as in Example 5 and exposed to UV light (260 nm). The DNA fragments are visible by fluorescence as bands arranged across the gel from the origin in order of decreasing size.

## Example 8

### Preparation of Labelled Probe

The labelling of probe DNA with $^{32}$P was performed using reagents supplied by Bethesda Research Labs Inc. (Gaithersburg, MD).

### Solution A

0.2 mM dATP

0.2 mM dGTP

0.2mM dTTP

500 mM Tris-HCl, pH 7.5

50  mM MgCl$_2$

100 mM 2-Mercaptoethanol

100 µg/ml Nuclease-free bovine serum albumin (BSA)

### Solution B

0.4 units /µl DNA polymerase I

40  pg/µl DNAse I

50 mM Tris-HCl, pH 7.5

5  mM Magnesium acetate

1  mM 2-Mercaptoethanol

0.1  mM Phenylmethylsulfonyl fluoride (PMSF)

50% Glycerol

100 µg/ml Nuclease-free BSA

The following reagents were added to a 1.5 ml capacity plastic centrifuge tube and mixed:

5 µl of Solution A

5 µl of solution containing 1 mg of the target
    DNA (to be labelled)

5 µl $^{32}$P-labelled dCTP (sp.act. 3000 Ci/mM)
    (Amersham Corp., Arlington, Heights, IL)

30 µl distilled water

Five µl of Solution B was added and the solution was again mixed and maintained at room temperature (18°C) for 2 hours. The reaction was stopped by the addition of 5 µl of a 300 mM EDTA solution.

The $^{32}$P-labelled probe DNA was separated from $^{32}$P-labelled dCTP on a small (0.5x5.0 cm) column of Sephadex G-50$^{TM}$ (Pharmacia Fine Chemicals, Piscataway, NJ) equilibrated with Tris-EDTA buffer (10 mM Tris, 1 mM EDTA, pH 8.0). The $^{32}$P-labelled probe was eluted in a volume of 0.3 ml and contained $1.2 \times 10^8$ cpm activity.

The hybridization mixture was prepared as described by Shinnick, T.M., E. Lund, O. Smithies, and F.R. Blattner, Nucl. Acids Res. 2:1911-1929 (1975). Into a glass test tube were added:

5.0 ml formamide (Fisher Scientific, Rockville,
    MD)

10 µl (10 mg) boiled sheared salmon testes DNA
    (P-L Biochemicals, Inc., Milwaukee, WI)

2.0 ml buffer (0.02 M Tris-HCl, 0.3 M KCl, 0.001
    M EDTA, pH 7.6)

27  ml distilled water

0.3 ml $^{32}$P-labelled probe DNA, prepared as
    above

The contents were mixed to provide a $^{32}$P-labelled probe solution suitable for hybridization with, and detection of, target DNA in test samples.

## Example 9
### Hybridization

A. All operations are performed at 42°C in hybridization/washing chamber 400:

Gel matrix 2 in handling frame 100 contains DNA fragments which have been separated according to size by electrophoresis in electrophoresis unit 200 (Example 7A). The gel matrix is dried in microwave/dryer 300 for 5 min. under microwave in vacuo as in Example 5 and placed in hybridization/washing unit 400. Probe reservoirs 444 are filled with probe prepared according to Example 8.

The DNA fragments in gel matrix 2 are denatured by filling chambers 404 in body 408 with 1 $\underline{M}$ KOH solution delivered via pump 426 and valve 420 from reservoir 412. Valve 452 is closed and overflow pipe 406 is set to maintain a level of 0.3 cm solution over the surface of gel matrix 2. The 1 $\underline{M}$ KOH solution is circulated at 10 ml/min. for 10 min.; then pump 426 shuts off, valve 420 closes and valve 452 opens to allow chambers 404 to drain into reservoir 450.

During denaturation the gel absorbs approximately 0.3-0.5 ml base solution and rehydrates. It is then washed for 15 min. with buffer (0.020 $\underline{M}$ Tris-HCl, 0.3 $\underline{M}$ KCl, 0.001 $\underline{M}$ EDTA, pH 7.6) from reservoir 410 (delivered via pump 426 and valve 418; valve 452 is closed) at a rate of 70 ml/min. for 20 min. to neutralize the base.

**0134622**

The neutralized gel is overlaid with a thin layer of probe delivered by micropump 440 via dispenser unit 430, 1 ml per channel 102, beginning 1 cm from the origin and traversing 13 cm down the length of gel 2.

After 3 hours, excess probe is washed from the gel by a flow of buffer from reservoir 410 at 10 ml/min. for at least 30-45 min. The gel frame now contains gel matrix 2 containing the sample DNA fragments (separated by electrophoresis in electrophoresis unit 200) hybridized to complementary $^{32}$P-labelled DNA probe. The configuration of separate bands produced by electrophoresis is retained. Approximately 0.001-0.01% of the probe activity ($0.2 \times 10^8$ dpm) is hybridized with target DNA in each channel 102 (200-2000 dpm) so that each separated complementary fragment has a minimum of 10 dpm radioactive material.

B. The DNA fragments are dyed by exposure for 10 min. to ethidium bromide from reservoir 414 (delivered via pump 426 and valve 422; valve 452 is closed). After 10 min. pump 426 stops, valve 422 closes, valve 452 opens and the dye drains from body 408. The gel matrix is then dried in microwave/dryer 300 under microwave in vacuo for 5 min. as in Example 5.

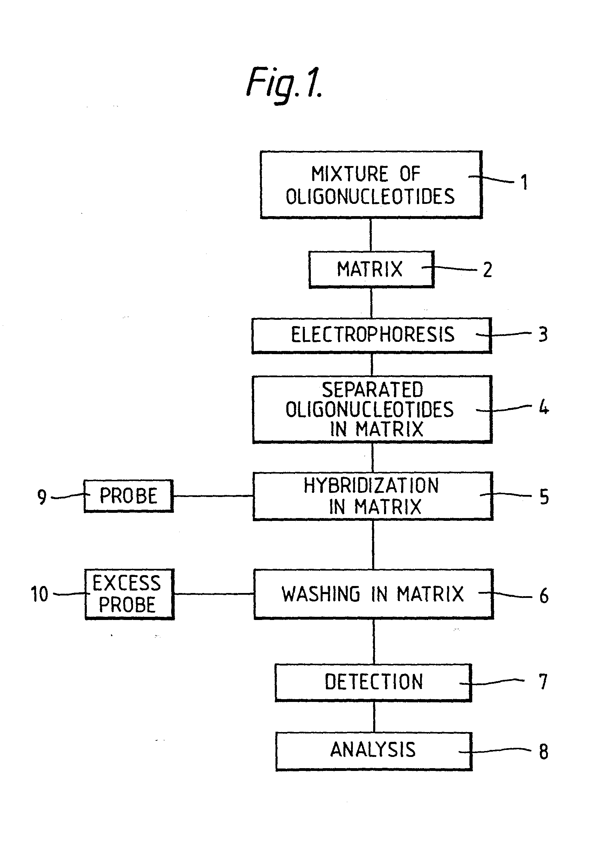

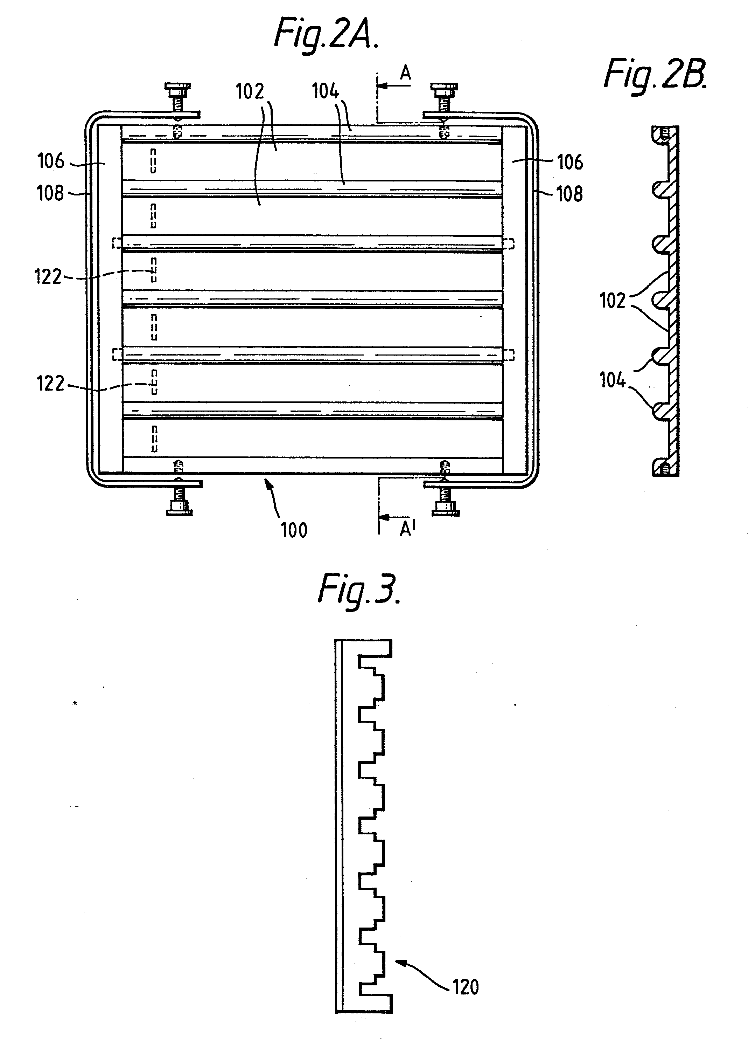

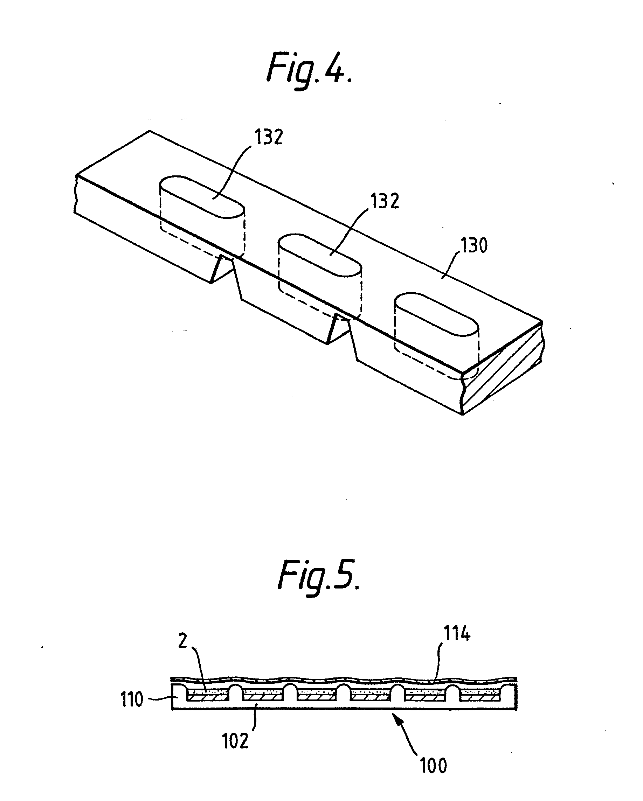

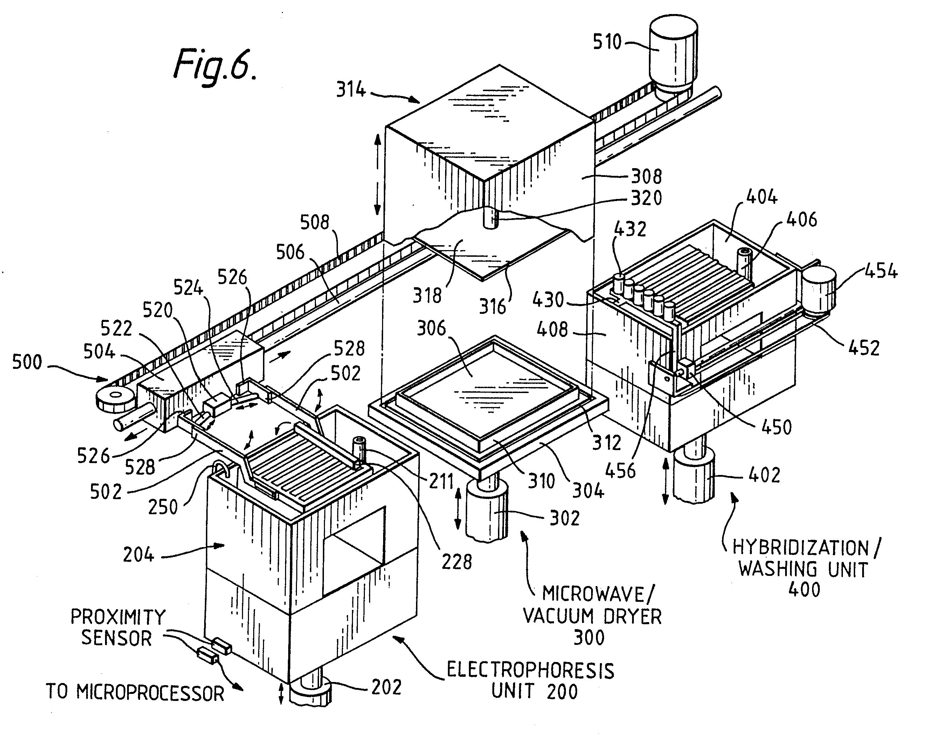

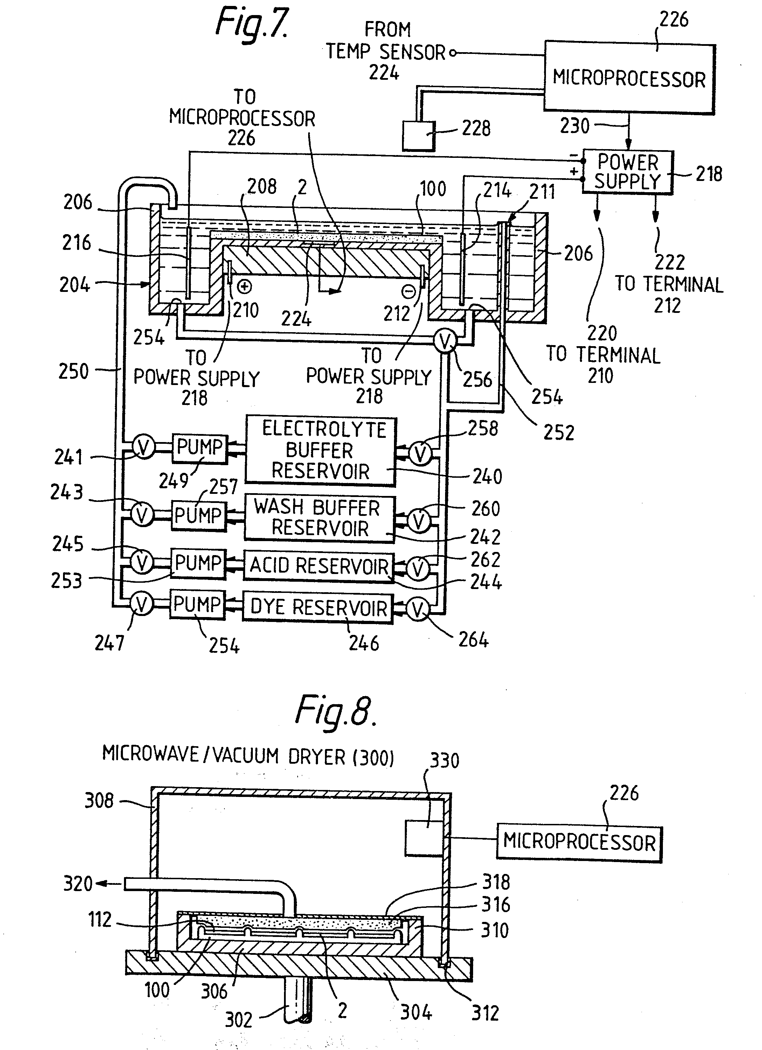

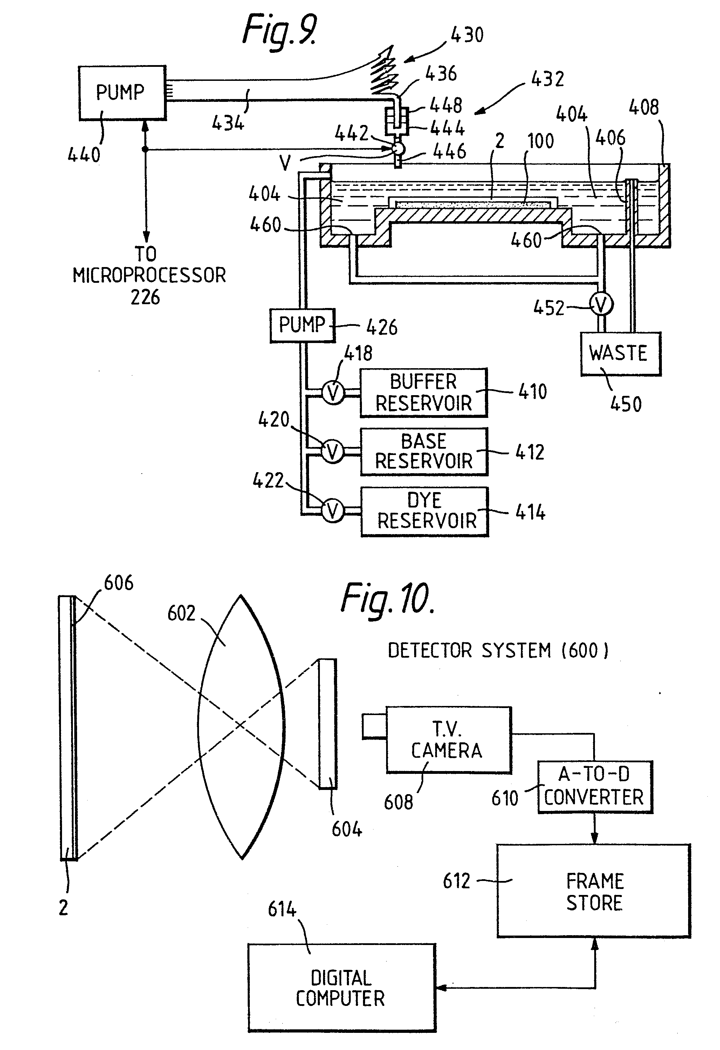

CLAIMS:

1. A system for the separation and identification of polynucleotides, said system comprising:

a) frame means for supporting a gel matrix containing a sample of polynucleotides,

b) transport means for moving said frame means along a predetermined path,

c) means in said path for separating by size and charge said sample of polynucleotides in said gel matrix, including means for applying potential across said frame means when said frame means is disposed therein by said transport means to cause electrophoretic action,

d) hybridization means disposed in said path and adapted to receive said frame means, said hybridization means comprising a hybridization chamber and a probe dispensing means adapted to apply probe solution onto said gel matrix, and

f) control means connected to said transport means, separation means, and hybridization means for actuating said electrophoresis means and hybridization means when said frame means is emplaced therein.

2. The system of Claim 1, further including sensor means for sensing the presence of radiative energy in said gel matrix thereby to detect the presence of hybridized polynucleotides therein.

3. The system of Claim 2, wherein said sensor means comprises means for sensing said radiative energy and converting said radiative energy to spatially defined electronic signals, and means for displaying said signals.

4. The system of Claim 1, wherein said separation means comprises:

a) chamber means adapted to contain an electrophoretic buffer solution and adapted to receive said frame means; said chamber means including a platform for supporting said frame means and electrode means for applying a potential across said frame means,

b) variable power supply means coupled to said electrode means,

c) means for sensing the temperature of said gel matrix, and

d) power control means responsive to said sensed temperature to vary the voltage applied to said electrode means to optimize the rate of electrophoresis.

5. The system of Claim 4 wherein said platform for supporting said frame means contains a temperature control platen connected to said variable power supply means; wherein the power supplied to said platen is regulated by said power control means in response to said sensed temperature.

6. The system of Claim 4, further including means for filling and draining said chamber means with electrophoretic buffer, wash buffer, acid solution, and/or dye solution from reservoirs.

7. The system of Claim 4, further including means movably disposed over said frame means for detecting the presence of a dye front and signalling

3

said presence to said control means to cause the power to be removed from said electrodes.

8. The system of Claim 1, wherein said probe dispensing means comprises a plurality of dispensing heads each containing a probe reservoir; said probe dispensing assembly being movably mounted on said hybridization chamber so as to enable said assembly to move and deposit probe along the length of said frame means.

9. The system of Claim 1, wherein said hybridization means further includes means for filling and draining of said hybridization chamber with wash buffer, base solution, and/or dye solution from reservoirs.

10. The system of Claim 1, further including a drying means disposed in said predetermined path for selectively drying said gel matrix under control of said control means.

11. A rapid system for the separation and identification of polynucleotides; said system comprising electrophoresis means for separating a mixture of polynucleotides according to charge and size in a gel matrix comprising about 0.2 to about 1.5 mm agarose; hybridization means for the in situ hybridization of said separated polynucleotides in said gel matrix with a complementary probe sequence, wherein either said polynucleotide mixture or said complementary probe sequence is a material labelled with a radiative energy-emitting marker; wash means for removing unhybridized labelled material after hybridization; and detector means for detecting the hybridized labelled material, said detector means comprising a scintillator plate adapted to be disposed over said gel matrix after hybridization, means for electrically scanning said plate to spatially determine points of light emanation and to create electrical signals indicative thereof, means for electronically storing said signals, and means for displaying said signals.

12. The system of Claim 11, further including microwave drying means for drying said gel matrix.

13. An apparatus for the electrophoretic separation of biopolymers, said apparatus comprising:

a)  chamber means adapted to contain an electrophoretic buffer solution and adapted to receive an electrophoretic gel matrix disposed on a solid inert backing means, said electrophoretic gel matrix having therein the biomolecules to be separated; said chamber means including a platform for supporting said backing means and electrode means for applying a potential across said gel matrix,

b)  variable power supply means coupled to said electrode means,

c)  means for sensing the temperature of said electrophoretic gel matrix, and

d)  control means responsive to said sensed temperature to vary the voltage applied to said electrode means to optimize the rate of electrophoresis.

14.  The apparatus of Claim 13, wherein said platform for supporting said backing means contains a temperature control platen connected to said variable power means; wherein the power supplied to said platen is regulated by said control means in response to said sensed temperature.

15. The apparatus of Claim 13, further including means for filling and draining said chamber means with electrophoretic buffer, wash buffer, acid solution, and/or dye solution from reservoirs.

16. The apparatus of Claim 13, further including a dye-front detector means movably disposed over said frame means for detecting the presence of a dye front and signalling said presence to said control means to cause the power to be removed from said electrodes.

17. An apparatus for the identification of the presence or absence of a specific polynucleotide sequence in a given set of polynucleotides in a gel matrix, said apparatus comprising:

    a)   a hybridization chamber, and

    b)   probe dispensing means adapted to apply probe solution onto said gel matrix.

18. The apparatus of Claim 17, wherein said probe dispensing means comprises a plurality of dispensing heads each containing a probe reservoir; said probe dispensing assembly being movably mounted on said hybridization chamber so as to enable said assembly to move and deposit probe along the length of said gel matrix.

19. The apparatus of Claim 17, further including means for filling and draining said hybridization chamber with wash buffer, base solution, and/or dye solution from individual reservoirs.

6

**0134622**

20. An apparatus for the detection of the presence of radiative energy in a gel matrix to indicate the presence of hybridized polynucleotides in said gel matrix, said apparatus comprising means for sensing said radiative energy and converting said radiative energy to spatially defined electronic signals, and means for displaying said signals.

21. A process for the detection of a specific target polynucleotide fragment in a mixture of polynucleotides, said process comprising:

a) electrophoretically separating said mixture of polynucleotides on a gel matrix which comprises about 0.2 to about 1.5 mm agarose gel, thereby producing within said gel a series of polynucleotide bands wherein each band represents polynucleotide sequences separated by size and charge,

b) adding to said polynucleotide bands which have been previously denatured a polynucleotide probe comprising a polynucleotide sequence the target polynucleotide whose presence or absence is to be determined, wherein either the target polynucleotide or the probe polynucleotide is labelled with a radiative energy-emitting material,

c) allowing the target polynucleotide to rest in contact with the probe polynucleotide until hybridization has occurred,

d) removing non-hybridized labelled polynucleotides, and

e) analyzing said gel matrix for the presence or absence of hybridized labelled polynucleotides.

22. The process of Claim 21, wherein said agarose gel comprises about 0.5 to about 2.0% (w/v) agarose.

# Fig.1.

```
                    ┌──────────────────┐
                    │    MIXTURE OF    │──── 1
                    │  OLIGONUCLEOTIDES│
                    └──────────────────┘
                            │
                    ┌──────────────────┐
                    │      MATRIX      │──── 2
                    └──────────────────┘
                            │
                    ┌──────────────────┐
                    │   ELECTROPHORESIS│──── 3
                    └──────────────────┘
                            │
                    ┌──────────────────┐
                    │    SEPARATED     │
                    │  OLIGONUCLEOTIDES│──── 4
                    │    IN MATRIX     │
                    └──────────────────┘
                            │
   ┌────────┐       ┌──────────────────┐
 9─│ PROBE  │───────│   HYBRIDIZATION  │──── 5
   └────────┘       │    IN MATRIX     │
                    └──────────────────┘
                            │
   ┌────────┐       ┌──────────────────┐
10─│ EXCESS │───────│  WASHING IN MATRIX│──── 6
   │ PROBE  │       └──────────────────┘
   └────────┘               │
                    ┌──────────────────┐
                    │    DETECTION     │──── 7
                    └──────────────────┘
                            │
                    ┌──────────────────┐
                    │     ANALYSIS     │──── 8
                    └──────────────────┘
```

**Fig.2A.**

**Fig.2B.**

**Fig.3.**

*Fig.4.*

132

132

130

*Fig.5.*

2

114

110

102

100

Fig.6.

PROXIMITY
SENSOR

TO MICROPROCESSOR

ELECTROPHORESIS
UNIT 200

MICROWAVE/
VACUUM DRYER
300

HYBRIDIZATION/
WASHING UNIT
400

4/6

0134622

0134622

## Fig.7.

## Fig.8.

MICROWAVE/VACUUM DRYER (300)

0134622

**Fig.9.**

- PUMP
- 430
- 436
- 432
- 448
- 444
- 446
- 442
- 434
- 440
- V
- 404
- 460
- 2
- 100
- 404
- 406
- 408
- 460
- TO MICROPROCESSOR 226
- PUMP — 426
- 452
- 450 WASTE
- 418
- BUFFER RESERVOIR — 410
- 420
- BASE RESERVOIR — 412
- 422
- DYE RESERVOIR — 414

**Fig.10.**

DETECTOR SYSTEM (600)

- 606
- 602
- 604
- 2
- T.V. CAMERA
- 608
- A-TO-D CONVERTER
- 610
- 612
- FRAME STORE
- 614
- DIGITAL COMPUTER